# EUROPEAN PATENT APPLICATION

(11) **EP 4 772 163 A1**
(43) Date of publication of application: **08.07.2026**
(21) Application number: 25382004.7
(22) Date of filing: 03.01.2025
(51) Int. Cl.: A61K 8/64, A61K 8/67, A61K 8/9722, A61Q 19/08

(54) **COSMETIC COMPOSITION FOR SKIN REJUVENATION**

(71) Applicant: Rofersam, S.A., 08520 Les Franqueses del Vallés (ES)
(72) Inventor: SANCHEZ FERNANDEZ, Juan Manuel, Vallromares (ES)
(74) Representative: Isern Patentes y Marcas S.L.

(57) **Abstract**

The invention relates to a cosmetic composition combining the Pal-Gly-His-Lys peptide, the Pal-Gly-Gln-Pro-Arg peptide, *Chlorella vulgaris* extract, niacinamide and at least one cosmetically acceptable excipient or adjuvant. The composition of the invention improves the appearance of skin, enhancing firmness, hydration and regeneration.

## Description

### Technical field

The present invention relates to a cosmetic composition comprising the peptides palmitoyl tripeptide-1, palmitoyl tetrapeptide-7, *Chlorella vulgaris extract and* niacinamide, to the use of the combination of active ingredients for the preparation of a cosmetic composition, and to the non-therapeutic use thereof in the treatment, prevention and/or repair of the signs of ageing and/or photoageing of the skin.

### Background of the invention

Skin ageing is a natural process influenced by intrinsic factors, such as decreased cellular activity and collagen production, and extrinsic factors, such as exposure to ultraviolet radiation, pollution, stress and lifestyle habits. These factors combined cause a series of visible skin changes, including the appearance of wrinkles, loss of firmness or spots, among others.

The main signs of skin ageing encompass a wide range of visible and structural skin changes. These include, among others, the appearance of wrinkles and fine lines, caused by a decrease in collagen and elastin, which are the fibres responsible for keeping the skin smooth and elastic. This loss of elasticity and firmness leads to a flabbier and less defined appearance. Furthermore, age spots and uneven pigmentation, resulting from the accumulation of sun damage over time, affect the even tone of the skin. Dryness and dehydration are also common, since skin loses its natural ability to retain moisture over the years, causing a feeling of roughness and lack of vitality.

Moreover, loss of volume, particularly in areas such as cheeks and lips, leads to a more aged appearance, while rough texture and enlarged pores reflect a decrease in the skin's regenerative capacity. Broken capillaries and redness, often associated with vascular fragility, create visible irregularities that affect the overall appearance. Finally, overall flabbiness, along with these signs, accentuates the sense of ageing by reducing definition in facial and body contours, reflecting the cumulative effects of intrinsic and extrinsic ageing factors.

There is no single cosmetic ingredient or medicinal product that can effectively address all signs of skin ageing. In response to this need, the present invention relates to an innovative cosmetic composition specifically designed to simultaneously treat, prevent and/or repair the various signs of skin ageing.

The invention described herein provides a comprehensive solution to this challenge, presenting an anti-ageing cosmetic composition that effectively acts on all visible signs of skin ageing, is free of irritant or toxic effects, and does not pose any risk to skin health.

### Summary of the invention

In a first aspect, the invention relates to a cosmetic composition comprising:
- palmitoyl tripeptide-1 (Pal-Gly-His-Lys);
- palmitoyl tetrapeptide-7 (Pal-Gly-Gln-Pro-Arg);
- *Chlorella vulgaris* extract;
- niacinamide; and
- at least one cosmetically acceptable excipient or adjuvant.

This innovative cosmetic composition offers a comprehensive solution for anti-ageing care of the skin by combining active ingredients that work synergistically to improve multiple signs of skin ageing. The cosmetic composition acts on the following signs of skin ageing:
- Wrinkles and fine lines: The peptides and niacinamide work synergistically to stimulate collagen and elastin production, reducing the appearance of wrinkles and fine lines.
- Loss of firmness and elasticity: The *Chlorella vulgaris* extract and peptides improve skin firmness and elasticity, providing a smoother, more youthful appearance.
- Age spots and uneven pigmentation: Niacinamide helps to reduce hyperpigmentation and age spots, making the skin tone even.
- Dryness and dehydration: The active ingredients help to keep the skin hydrated, improving its softness and texture.
- Loss of volume: The formulation contributes to the improvement of skin density and volume, providing a fuller, more youthful appearance.
- Rough texture: Cell renewal promoted by the active ingredients improves skin texture, making it smoother and more even.
- Enlarged pores: Niacinamide helps to reduce the appearance of enlarged pores, improving overall skin texture.
- Broken capillaries and redness: The anti-inflammatory properties of niacinamide and the *Chlorella vulgaris* extract help to reduce redness and the appearance of broken capillaries.
- General flabbiness: The combination of active ingredients improves skin firmness and elasticity, reducing flabbiness and providing a more toned appearance.

In a second aspect, the invention relates to the cosmetic composition according to the first aspect of the invention, for the non-therapeutic use thereof in the treatment, prevention and/or repair of the signs of ageing and/or photoageing of the skin.

In a third aspect, the invention relates to the use the combination of the Pal-Gly-His-Lys peptide, the Pal-Gly-Gln-Pro-Arg peptide, *Chlorella vulgaris* extract and niacinamide in the preparation of a cosmetic composition.

### Detailed description of the invention

All the terms used in this application, unless otherwise indicated, shall be understood according to their common meaning in the corresponding technical field. However, more specific definitions of certain terms used in the present application are set forth below and intended to be applied uniformly in the entire specification and claims, unless an expressly established definition indicates a broader meaning.

In the present document, the indefinite articles "a" and "an" are synonymous with "at least one" or "one or more". Unless otherwise indicated, the definite articles used in this document, such as "the", also include the plural of the noun.

The present invention provides a cosmetic composition comprising:
- Pal-Gly-His-Lys;
- Pal-Gly-Gln-Pro-Arg;
- *Chlorella vulgaris* extract;
- niacinamide; and
- at least one cosmetically acceptable excipient or adjuvant.

The expression "cosmetically acceptable" refers to components that must be acceptable in the sense of being compatible with the other ingredients of the composition. Furthermore, it must be suitable for use in contact with the without presenting excessive toxicity, irritation, allergic response, immunogenicity or other problems or complications that exceed a reasonable benefit/risk balance.

The peptides Pal-Gly-His-Lys (the nomenclature of which according to the IUPAC is (2S)-6-amino-2-[[(2S)-2-[[2-(hexadecanoylamino)acetyl]amino]-3-(1H-imidazol-5-il)propanoyl]amino]hexanoic acid and having CAS number 147732-56-7) and Pal-Gly-Gln-Pro-Arg (with the IUPAC nomenclature, (2S)-2-[[(2S)-1-[(2S)-5-amino-2-[[2-(hexadecanoylamino)acetyl]amino]-5-oxopentanoyl]pyrrolidin-2-carbonyl]amino]-5-(diaminomethylideneamino)pentanoic acid and having CAS number 221227-05-0) stimulate collage and elastic production, reducing the appearance of wrinkles and fine lines, improving skin firmness and elasticity, and helping to maintain hydration. Said peptides have the following chemical formula:

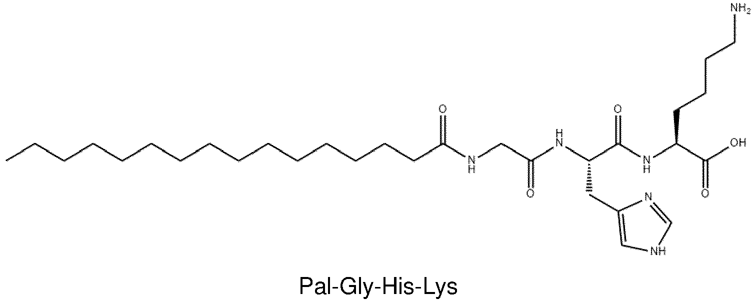

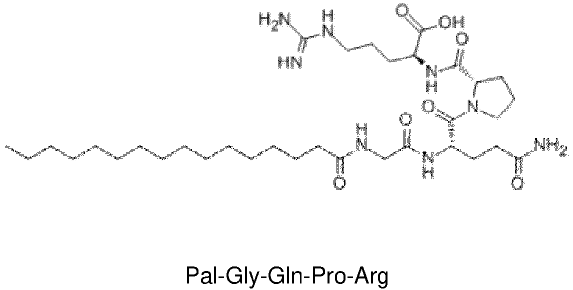

In the context of the present invention, "skin" is understood as the set of layers making up same from the most superficial layer or stratum corneum to the deepest layer or hypodermis, both included. Said layers are made up of different types of cells such as, for example, keratinocytes, fibroblasts, melanocytes and/or adipocytes, among others. In the context of the present invention, the term skin comprises the scalp.

In the present description, the abbreviations used for amino acids follow the rules of the Biochemical Nomenclature Commission of the IUPAC-IUB specified in Eur. J. Biochem. (1984) 138:9-37.

In this way, for example, Ala represents NH₂-CH(CH₃)-COOH, Ala- represents NH₂-CH(CH₃)-CO-, -Ala represents -NH-CH(CH₃)-COOH and -Ala- represents -NH-CH(CH₃)-CO-. Therefore, the dash, which represents a peptide bond, removes OH from the 1-carboxyl group of the amino acid (represented herein in the conventional non-ionised form) when it is located to the right of the symbol, and removes H from the 2-amino group of the amino acid when it is located to the left of the symbol; both modifications can be applied to the same symbol. The abbreviation "Palm-" is used to designate the palmitoyl group (CH₃-(CH₂)₁₄-CO-).

The term "extract" refers to a substance obtained when isolating bioactive components of plant, animal, mineral or marine origin by means of an extraction process. This process may include techniques such as maceration, percolation, distillation or solvent extraction, among others.

*Chlorella vulgaris* is a unicellular, deep green microalgae, recognised for its exceptional nutritional content and its wide versatility in various applications. It is a rich source of protein (50-60% of its dry weight), essential amino acids, vitamins such as B12, minerals and potent antioxidants that promote collagen and elastin synthesis. Its extract (CAS 223749-83-5) offers significant benefits to the skin, helping to protect it from oxidative damage, stimulate cell renewal and reduce inflammation. This translates into firmer, more elastic skin with a texture that is visibly healthier and more even.

Niacinamide (pyridine-3-carboxamide, CAS number 98-92-0), also known as vitamin B3, is a versatile and highly effective skin care ingredient valued for its multiple benefits. It acts by improving the appearance of wrinkles and fine lines, visibly reducing hyperpigmentation and promoting greater skin elasticity. Furthermore, it is known for its powerful anti-inflammatory properties, which soothe sensitive or irritated skin, and for its ability to strengthen the skin barrier, helping to maintain hydration and protect against external agents. It also regulates sebum production, making it ideal for oily or acne-prone skin, while minimising the appearance of enlarged pores. Its antioxidant action combats damage caused by free radicals, contributing to a more luminous, even and healthy skin.

The synergy between these 4 components not only addresses the signs of ageing in a comprehensive manner, but it also enhances their individual effects, offering an advanced and effective skin care solution. This formulation represents a significant advancement in antiageing cosmetics, providing visible and long-lasting benefits to achieve younger and healthier skin.

In the context of this invention, the term "ageing" refers to changes in the skin due to the passage of time (chronoageing), sun exposure (photoageing) or environmental factors such as cigarette smoke, extreme weather conditions (cold or wind), chemical pollutants and pollution. These changes include all alterations that are visible or perceptible by touch such as wrinkles, fine lines, stretch marks, striae, cracks, textural irregularities, increased pore size, loss of hydration, elasticity, firmness, smoothness, resilience and recovery capacity, as well as sagging in areas such as cheeks, bags under the eyes or double chin. It also comprises alterations in colouration, such as spots, redness, dark circles under the eyes and hyperpigmented areas (age spots or freckles), as well as hyperkeratinisation, elastosis, keratosis, hair loss, orange peel appearance, degradation of the collagen structure and other histological changes in the stratum corneum, dermis, epidermis, vascular system (appearance of spider veins or telangiectasias) and tissues close to the skin.

The term "photoageing" refers to the set of processes caused by prolonged exposure of the skin to ultraviolet radiation, leading to premature ageing of the skin. This process shares the physical characteristics of natural ageing, such as flabbiness, sagging, colour changes, pigmentation irregularities and abnormal and/or excessive keratinisation, among others. Furthermore, exposure to environmental factors such as cigarette smoke, pollution and adverse weather conditions (such as cold and/or wind) also lead to skin ageing.

In another embodiment of the first aspect of the invention, the cosmetic composition comprises a concentration of the Pal-Gly-His-Lys peptide of between 0.00005% and 0.00050% by weight with respect to the composition, preferably between 0.00015% and 0.00035%, more preferably between 0.00025% and 0.00035%.

In another embodiment of the first aspect of the invention, the cosmetic composition comprises a concentration of the Pal-Gly-Gln-Pro-Arg peptide of between 0.00005% and 0.00025% by weight with respect to the composition, preferably between 0.00010% and 0.00020%.

In another embodiment of the first aspect of the invention, the cosmetic composition comprises the Pal-Gly-Gln-Pro-Arg and Pal-Gly-His-Lys peptides in a weight ratio of between 1:1 and 1:5, more preferably between 1:1 and 1:3.

The "weight ratio" between two or more compounds refers to the ratio of mass or weight, in other words, the proportion between the weight (in mass units) of the compounds and the total weight of the composition.

In another embodiment of the first aspect of the invention, the cosmetic composition comprises a concentration of the *Chlorella vulgaris* extract of between 0.50% and 2.5% by weight with respect to the composition, preferably between 1% and 2%.

In another embodiment of the first aspect of the invention, the cosmetic composition comprises a concentration of niacinamide of between 5 and 15% by weight with respect to the composition, preferably between 8% and 12%, preferably between 9% and 11%.

In a preferred embodiment of said first aspect of the invention, the concentration of the Pal-Gly-His-Lys peptide is between 0.00005% and 0.00050% by weight with respect to the composition, the concentration of the Pal-Gly-Gln-Pro-Arg peptide is between 0.00005% and 0.00025% by weight with respect to the composition, the concentration of the *Chlorella vulgaris* extract is between 0.50% and 2.5% by weight with respect to the composition, and the concentration of niacinamide is between 5 and 15% by weight with respect to the composition.

In an embodiment of the first aspect of the invention, compatible with any of the preceding embodiments, the at least one cosmetically acceptable excipient or adjuvant is selected from a surfactant, an emulsifier, a thickener, a preservative, a perfume, a pH regulator, or mixtures thereof.

Compositions for topical or transdermal application can be formulated in a wide variety of solid, liquid or semisolid presentations including, but not limited to, creams, multiple types of emulsions, such as oil and/or silicone in water emulsions, water in oil and/or silicone emulsions, water/oil/water, water/silicone/water, oil/water/oil or silicone/water/silicone type emulsions. They can also be presented as anhydrous compositions, aqueous dispersions, oils, milks, balsams, foams, lotions, gels, cream gels, hydroalcoholic solutions, hydroglycolic solutions, hydrogels, liniments, sera, serums, polysaccharide films, ointments, mousses or salves. In the preferred embodiment of the invention, it is formulated as an emulsion in water.

The compositions of the invention can be prepared by means of the conventional methods known to those skilled in the art ("Harry's Cosmeticology", Seventh edition, (1982), Wilkinson J.B., Moore R.J., ed. Longman House, Essex, GB).

In an embodiment of the first aspect of the invention, the cosmetic composition is used with non-therapeutic purposes for the treatment, prevention and/or repair of the signs of ageing and/or photoageing of the skin.

The second aspect of the invention relates to the use of the combination of Pal-Gly-His-Lys, Pal-Gly-Gln-Pro-Arg, *Chlorella vulgaris* extract and niacinamide in the preparation of a cosmetic composition.

Throughout the description and claims, the word "comprise" and variations thereof are not intended to exclude other technical features, additives, components or steps. Furthermore, the word "comprise" includes the case of "consist of". Additional objects, advantages and features of the invention will be apparent to those skilled in the art. Additional objects, advantages and features of the invention will be apparent to those skilled in the art upon examining the description or can be learned by putting the invention into practice.

The following examples are provided by way of illustration and do not seek to limit the present invention. Furthermore, the present invention covers all the possible combinations of the particular and preferred embodiments described in this document.

### Examples

### Example 1. Preparation of a serum-type cosmetic composition

A specific embodiment of the composition of the invention, particularly, in the format of a serum, is described below.

**Table 1. Detail of the serum formulation.**

| **Component** | **Phase** | **% (w/w)** | **Role** |
|---|---|---|---|
| **Niacinamide** | A | 10.0 | Active ingredient |
| Propanediol | A | 6.60 | Solvent, moisturiser |
| Caprylic/Capric triglyceride | C | 6.50 | Emollient, skin conditioner |
| Glycerin | B | 4.56 | Moisturiser |
| Hydrogenated ethylhexyl olivate | C | 1.28 | Emollient |
| *Olea europaea* fruit oil | C | 0.50 | Conditioner |
| Sodium stearoyl glutamate | C | 0.15 | Emulsifier |
| *Chlorella vulgaris* extract | F | 0.13 | Active ingredient |
| Polyglycerin-3 | C | 0.008 | Moisturiser |
| **Palmitoyl tetrapeptide-7** | F | 0.00015 | Active ingredient |
| *Helianthus annuus* seed wax | C | 0.15 | Skin conditioner, thickener |
| Tocopherol | D | 0.09 | Antioxidant, skin conditioner |
| Glycine soja oil | D | 0.01 | Hydrating agent/Antioxidant |
| **Palmitoyl tripeptide-1** | F | 0.0003 | Active ingredient |
| Lecithin | F | 0.01 | Emulsifier, emollient |
| Hydrogenated unsaponifiables from olive oil | C | 0.22 | Conditioner |
| Mica | G | 0.343 | Opacifier |
| Jojoba esters | C | 0.357 | Emollient, hydrating agent, skin conditioner |
| Squalene | C | 0.5 | Hydrating agent, skin conditioner |
| Sclerotium gum | B | 0.6 | Gelling agent, skin conditioner |
| Carbomer | F | 0.03 | Gelling agent |
| Glyceryl stearate | C | 0.357 | Emulsifier, emollient |
| Xanthan gum | B | 0.20 | Thickener |
| Cetearyl alcohol | C | 0.62 | Emollient, emulsifier |
| Sodium lactate | F | 0.03 | pH regulator |
| Lactic acid | F | 0.004 | pH regulator |
| Butylene glycol | F | 0.6 | Moisturiser |
| Citric acid | H | 0.06 | pH regulator |
| 1,2-hexanediol | E | 0.25 | Solvent |
| Caprylyl glycol | E | 0.25 | Moisturiser, emollient, skin conditioner |
| Hydroxyacetophenone | A | 0.5 | Antioxidant |
| Perfume | I | 0.15 | Aromatic substance |
| Titanium dioxide | G | 0.35 | Sunscreen |
| Caramel | J | 0.158 | Colourant |
| Water | A | q.s. | Solvent |

The formula described above was prepared following the method described below.
i.) Heating the components of phase A and phase C at 75-80°C.
ii.) Mixing the ingredients of phase B with a blade and adding them directly to the vortex of phase A, using turbine stirring at medium/high speed and blade at medium speed for 20-25 minutes or until obtaining a homogeneous mixture.
iii.) Leaving it to rest for several minutes to allow the release of air.
iv.) Before performing emulsification, combining the ingredients of phase D with those of phase C once the latter reaches 75°C. Stirring with a blade at medium speed for 2 minutes.
v.) Ensuring that the mixtures (A+B) and (C+D) are at a temperature of 75-80°C before performing emulsification.
vi.) Performing emulsification by adding (C+D) to (A+B) using turbine stirring at medium speed and blade at medium speed for 15 minutes.
vii.) Incorporating phase E under turbine stirring at medium speed and blade at low speed for 5 minutes.
viii.) Continuing to stir with the blade at medium speed until the emulsion is homogeneous.
ix.) When the temperature drops below 40°C, adding the ingredients of phase F, one by one, followed by phases G, H and I. Stirring using turbine at medium speed and blade at low speed for 5 minutes after each addition.
x.) Adjusting the colour, where necessary, using the ingredients of phase J.

## Claims

1. A cosmetic composition comprising:
- Pal-Gly-His-Lys;
- Pal-Gly-Gln-Pro-Arg;
- *Chlorella vulgaris* extract;
- niacinamide; and
- at least one cosmetically acceptable excipient or adjuvant.

2. The cosmetic composition according to the preceding claim, wherein the concentration of the Pal-Gly-His-Lys peptide is between 0.00005% and 0.00050% by weight with respect to the composition, preferably between 0.00015% and 0.00035%, more preferably between 0.00025% and 0.00035%.

3. The cosmetic composition according to any of the preceding claims, wherein the concentration of the Pal-Gly-Gln-Pro-Arg peptide is between 0.00005% and 0.00025% by weight with respect to the composition, preferably between 0.00010% and 0.00020%.

4. The cosmetic composition according to any of the preceding claims, wherein the concentration of the *Chlorella vulgaris* extract is between 0.50% and 2.5% by weight with respect to the composition, preferably between 1% and 2%.

5. The cosmetic composition according to any of the preceding claims, wherein the concentration of niacinamide is between 5 and 15% by weight with respect to the composition, preferably between 8% and 12%, preferably between 9% and 11%.

6. The cosmetic composition according to any of the preceding claims, wherein the at least one cosmetically acceptable excipient or adjuvant is selected from a surfactant, an emulsifier, a thickener, a preservative, a perfume, a pH regulator, or mixtures thereof.

7. The cosmetic composition according to any of claims 1 to 6, for the non-therapeutic use thereof in the treatment, prevention and/or repair of the signs of ageing and/or photoageing of the skin.

8. Use of a combination of Pal-Gly-His-Lys, Pal-Gly-Gln-Pro-Arg, *Chlorella vulgaris* extract and niacinamide in the preparation of a cosmetic composition.

## Amended claims

### Amended claims in accordance with Rule 137(2) EPC.

1. A cosmetic composition comprising:
- Pal-Gly-His-Lys;
- Pal-Gly-Gln-Pro-Arg;
- *Chlorella vulgaris* extract;
- niacinamide in a concentration of between 5 and 15% by weight with respect to the composition; and
- at least one cosmetically acceptable excipient or adjuvant.

2. The cosmetic composition according to the preceding claim, wherein the concentration of the Pal-Gly-His-Lys peptide is between 0.00005% and 0.00050% by weight with respect to the composition, preferably between 0.00015% and 0.00035%, more preferably between 0.00025% and 0.00035%.

3. The cosmetic composition according to any of the preceding claims, wherein the concentration of the Pal-Gly-Gln-Pro-Arg peptide is between 0.00005% and 0.00025% by weight with respect to the composition, preferably between 0.00010% and 0.00020%.

4. The cosmetic composition according to any of the preceding claims, wherein the concentration of the *Chlorella vulgaris* extract is between 0.50% and 2.5% by weight with respect to the composition, preferably between 1% and 2%.

5. The cosmetic composition according to any of the preceding claims, wherein the concentration of niacinamide is between 8% and 12%, preferably between 9% and 11%.

6. The cosmetic composition according to any of the preceding claims, wherein the at least one cosmetically acceptable excipient or adjuvant is selected from a surfactant, an emulsifier, a thickener, a preservative, a perfume, a pH regulator, or mixtures thereof.

7. Use of the cosmetic composition according to any of claims 1 to 6, for the non-therapeutic treatment, prevention and/or repair of the signs of ageing and/or photoageing of the skin.

8. Use of a combination of Pal-Gly-His-Lys, Pal-Gly-Gln-Pro-Arg, *Chlorella vulgaris* extract and niacinamide in a concentration of between 5 and 15% with respect to the composition in the preparation of a cosmetic composition.
